Europäisches Patentamt

European Patent Office

Office européen des brevets

(19)

(11) Veröffentlichungsnummer: **0 145 980**
A2

# (12) EUROPÄISCHE PATENTANMELDUNG

(21) Anmeldenummer: 84113889.4

(22) Anmeldetag: 16.11.84

(51) Int. Cl.⁴: **C 07 C 103/46**, C 07 C 102/00

(30) Priorität: 15.12.83 DE 3345411

(43) Veröffentlichungstag der Anmeldung: **26.06.85**
**Patentblatt 85/26**

(84) Benannte Vertragsstaaten: **DE FR GB IT**

(71) Anmelder: **DYNAMIT NOBEL AKTIENGESELLSCHAFT,**
**Postfach 1261, D-5210 Troisdorf, Bez. Köln (DE)**

(72) Erfinder: **E-Chahawi, Moustafa, Dr., Pacellistrasse 4,**
**D-5210 Troisdorf (DE)**
Erfinder: **Meyer, Günter, Dr., Augustinusstrasse 6,**
**D-5210 Troisdorf (DE)**

(54) Verfahren zur Reindarstellung von Carbonsäuren.

(57) Aus Reaktionslösungen von Carbonsäuren, die durch Carbonylierung mit Hilfe von Kobaltcarbonyl-Katalysatoren hergestellt wurden, wird das Kobalt des Katalysators durch Zugabe von mindestens äquivalenten Mengen von Mono- oder Dicarbonsäuren und Neutralisation mit alkalischen Mitteln als Kobalt-II-Salz der zugegebenen Säure gefällt und die Carbonsäure der Reaktionslösung durch Ansäuern vollständig gewonnen.

EP 0 145 980 A2

Troisdorf, 5. Dez. 1983
OZ 83069 (4300) Dr.La./cp

DYNAMIT NOBEL AKTIENGESELLSCHAFT
Troisdorf Bez. Köln

Verfahren zur Reindarstellung von Carbonsäuren

Die Erfindung betrifft ein Verfahren zur Reindarstellung von Carbonsäuren aus ihren Reaktionslösungen, worin Kobaltcarbonyl-Katalysatoren wie insbesondere $Co_2(CO)_8$ oder entsprechende ionische Kobaltcarbonylverbindungen enthalten sind. Solche Kobaltcarbonyl-Katalysatoren enthalten den wertvollen Rohstoff Kobalt, der nach Möglichkeit wiedergewonnen werden soll. Weiterhin erschweren die Kobalt-Katalysatoren die Reindarstellung der Carbonsäuren und bilden Verunreinigen in den Zielprodukten.
Nach bekannten Verfahren der Carbonylierung mit Hilfe von Kobaltcarbonyl-Katalysatoren, insbesondere Dikobaltoctacarbonyl, können eine Reihe von Carbonsäuren durch Einfügung von einem oder ggf. zwei Molekülen CO und ggf. weiteren Molekülteilen hergestellt werden, wie beispielsweise Arylessigsäuren, besonders Phenylessigsäure, Malonsäure und deren Ester, Phenylbrenztraubensäure und zahlreiche weitere. Hierbei sind vergleichsweise geringe Mengen des Kobalt-Katalysators abzutrennen.
Im Falle der Herstellung von Acetyl-amino-carbonsäuren, insbesondere N-Acetyl-DL-phenylalanin werden jedoch sehr große Mengen von Koblatcarbonyl-Katalysator benötigt.

- 2 -

0145980

Insbesondere bezieht sich daher die Erfindung auf ein Verfahren der Reindarstellung von N-Acetyl-aminosäuren sowie unter diesen besonders von N-Acetyl-DL-phenylalanin durch Entfernung von Kobaltcarbonyl-Katalysatoren aus der Reaktionslösung unter gleichzeitiger Gewinnung des enthaltenen Kobalts.

N-Acetyl-aminocarbonsäuren, besonders N-Acetyl-$\alpha$-aminocarbonsäuren können auf an sich bekannte Weise nach der DE-OS 23 64 039 durch Umsetzung aus den entsprechenden organischen Halogeniden mit Wasserstoff und Kohlenmonoxid in Gegenwart von Kobaltcarbonyl-Katalysatoren und einem Amid hergestellt werden. Bei dieser Verfahrensweise sind große Mengen von Kobaltcarbonyl-Katalysatoren erforderlich. In der DE-OS wird jedoch kein technisch brauchbarer Weg angegeben, wie die großen Mengen des Katalysators selektriv und vollständig entfernt und das Kobalt gewonnen wird. Weiterhin gibt die DE-OS nicht an, wie die Acetylamino-carbonsäuren isoliert werden und in erforderlicher Weise von den Resten des Kobalts getrennt werden können. Demgemäß ist das Verfahren der DE-OS in der bekannten Form nicht verwertbar.

Es bestand die Aufgabe, Carbonsäuren aus ihren Reaktionslösungen mit Gehalten von Kobaltcarbonyl-Katalysatoren zu isolieren und nach Möglichkeit von Resten von Kobalt-Verbindungen zu befreien und das in den Kobaltcarbonyl-Katalysatoren enthaltene Kobalt in einer Form zu gewinnen, die eine erneute Verwendung des Kobalts ermöglicht.

Insbesondere bestand die Aufgabe darin, N-Acetyl-$\alpha$-aminocarbonsäuren und von diesen besonders N-Acetyl-DL-phenylalanin aus seinen Reaktionslösungen mit Gehalten von Kobaltcarbonyl-Katalysatoren rein und mit möglichst geringen Verlusten darzustellen sowie das Kobalt des Katalysators vom Zielprodukt zu trennen, aus der Reaktionslösung nach Möglichkeit vollständig zu entfernen und in wiedergewinnbarer Form zu isolieren.

Diese Aufgabe wird durch die Anforderung höher Reinheit für die Acetyl-aminosäuren erschwert, insbesondere soweit

0145980

diese für pharmazeutische Zwecke dienen sollen und insbesondere soweit diese als Ausgangsstoffe einer enzymatischen Racemat-Trennung vorgesehen sind. Weiterhin wird die Aufgabe erschwert durch unvollständige Bildung der N-Acetyl-aminocarbonsäure und hohe Anteile von Nebenprodukten sowie große Mengen Chloriden in der Reaktionslösung.

Es wurde gefunden, daß eine hohe Ausbeute und Reinheit der N-Acetyl-aminocarbonsäuren und insbesondere von N-Acetyl-DL-phenylalanin erreicht wird und daß eine Zerstörung und Umwandlung des Kobaltcarbonyl-Katalysators in der Reaktionslösung möglich ist und Kobalt als Salz einer zugegebenen Mono- oder Dicarbonsäure ausgefällt werden kann. Aus der dann entstehenden Lösung des Zielproduktes kann die hergestellte Carbonsäure, insbesondere N-Acetyl- -aminocarbonsäure und vorzugsweise N-Acetyl-DL-phenylalanin in rein Form mit guter Ausbeute gewonnen werden. Hierzu wird vorgeschlagen, das Zielprodukt in seine Salzform mit Basen zu überführen, durch Ansäuern mit konzentrierten Säuren, insbesondere Mineralsäuren, auszufällen und zu isolieren.

Gegenstand der Erfindung ist daher ein Verfahren zur Reindarstellung von Carbonsäuren aus Reaktionslösungen mit Gehalten von Kobaltcarbonyl-Katalysatoren, dadurch gekennzeichnet, daß man die Reaktionslösung

a) mit Monocarbonsäuren mit 1 bis 3 C-Atomen, Dicarbonsäuren mit 2 bis 3 C-Atomen oder deren wässrige Lösungen in mindestens äquivalenter Menge zum Kobalt des Katalysators versetzt

b) die Reaktionslösung durch Zugabe alkalischer Mittel neutralisiert und das Kobaltsalz der nach a) zugegebenen Säure entfernt, und

c) die Carbonsäure aus der Reaktionslösung aufarbeitet.

0145980

Weiterer Gegenstand der Erfindung ist ein Verfahren zur Aufarbeitung solcher Reaktionslösungen, dadurch gekennzeichnet, daß die organische Phase nach Neutralisation mit alkalischen Mitteln von der wässrigen Phase, welche das Zielprodukt enthält, getrennt wird.

Vorzugsweise wird dabei aus der entstehenden wässrigen Phase das Zielprodukt durch Ansäuern mit Säuren, insbesondere Mineralsäuren, gefällt und durch Abtrennung isoliert.

Weiterhin und vorzugsweise ist es von erheblichem Vorteil, die abgetrennte organische Phase erfindungsgemäß in den Herstellprozeß zurückzuführen. Zweckmäßig wird hierzu die organische Phase destilliert. Hierdurch werden besonders die verwendeten Lösungsmittel erneut der Herstellung der jeweiligen Carbonsäure zugänglich gemacht. Hierdurch werden besonders die verwendeten Lösungsmittel erneut der Herstellung der jeweiligen Carbonsäure zugänglich gemacht.

Es hat sich gezeigt, daß die bei der Carbonylierung entstandene Carbonsäure, insbesondere N-Acetyl-aminocarbonsäuren und von diesen besonders N-Acetyl-DL-phenylalanin, deren Gehalt analytisch in der Reaktionslösung feststellbar ist, praktisch quantitativ durch das vorliegende Verfahren der Reindarstellung gewonnen werden kann. Sehr bemerkenswert ist weiterhin die hohe Reinheit des Zielprodukts, insbesondere von N-Acetyl-DL-aminocarbonsäuren von 98 % oder mehr. Das jeweilige Produkt hat praktisch die theoretische Säurezahl.

Es wurde überraschend gefunden, daß das Kobalt aus dem nullwertigen Zustand in der organischen Phase der Reaktionslösung bzw. aus dem (-1)-wertigen Zustand in der wässrigen Phase praktisch quantitativ als 2-wertiges Kobaltsalz der zur Fällung verwendeten organischen Säure entfernt werden kann.

Zur Fällung können organische Carbonsäuren, insbesondere Monocarbonsäuren mit 1 bis 3 C-Atomen und von diesen besonders die gesättigten Monocarbonsäuren sowie Dicarbonsäuren mit 2 bis 3 C-Atomen insbesondere die gesättigten Dicarbonsäuren verwendet werden. Bevorzugt sind Ameisensäure und Oxalsäure.

Sehr bevorzugt ist die Oxalsäure.

Die zur Fällung verwendeten Carbonsäuren sollen in mindestens äquivalenter Menge, bezogen auf das Kobalt des Katalysators, das durch Analysen feststellbar ist, verwendet werden. Zweckmäßig ist ein Überschuß von bis zu 20 % über die äquivalente Menge, bevorzugt ein Überschuß von bis zu 10 %. Die verwendeten Säuren können als reine Stoffe in flüssiger oder fester Form oder als ihre wässrigen Lösungen zugegeben werden. Wässrige Lösungen werden zweckmäßig in möglichst konzentrierter Form zugegeben. Eine Zugabe in die noch heiße Reaktionslösung ist möglich.

Im allgemeinen wird die Zugabe bei Temperaturen von 20 bis $90^{0}C$, vorzugsweise bei 50 bis $80^{0}C$, erfolgen.

Vorzugsweise wird die Reaktionslösung nach der Zugabe der Mono- oder Dicarbonsäuren auf die Zugabetemperatur bis zu einer Stunde gehalten.

Danach wird durch alkalische Mittel, d.h. durch Zugabe von Alkalihydroxyd wie NaOH oder KOH oder Alkalicarbonate wie Natriumcarbonat, Natriumbicarbonat oder Kaliumcarbonat oder durch Zugabe von Calciumhydroxyd, bevorzugt in konzentrierter wässriger Lösung, die Reaktionslösung auf pH-Werte zwischen 5,8 bis 8, vorzugsweise 6,8 bis 7,2, eingestellt.

Das ausfallende Kobaltsalz der zugegebenen Mono- oder Dicarbonsäure kann darauf, beispielsweise durch Filtration, entfernt werden.

Nach der Filtration des Kobaltsalzes erhält man eine wässrige Phase mit dem Salz der Carbonsäure der Reaktionslösung, im Falle von N-Acetyl-$\alpha$-aminocarbonsäuren deren Salze, sowie eine organische Phase mit insbesondere darin enthaltenen Lösungsmitteln der Reaktionslösung der Carbonylierung sowie evtl. entstandenen Nebenprodukten.

Es hat sich gezeigt, daß eine ausgezeichnete Abtrennung des Zielproduktes von den übrigen Stoffen auf diese Weise erreichbar ist.

Die abgetrennte wässrige Phase kann nunmehr mit konzentrierten Säuren, insbesondere Mineralsäuren, wie Salzsäure, Schwefelsäure oder Phosphorsäure, sehr bevorzugt Salzsäure, zur vollständigen Ausfällung der durch Carbonylierung entstandenen Carbonsäure versetzt werden. Hierbei ist auf pH 1 anzusäuern. Wenn erforderlich kann bei oder nach Zusatz der Mineralsäure gekühlt werden. es zeigte sich, daß eine einwandfreie Abtrennung der durch Carbonylierung entstandenen Carbonsäure möglich ist. Insbesondere sind

N-Acetyl-$\alpha$-aminocarbonsäuren und von diesen insbesondere N-Acetyl-DL-phenylalanin in hoher Reinheit und mit außerordentlich geringen Kobaltgehalten sowie sehr vollständig aus der wässrigen Phase mit Zugabe der Säuren abtrennbar.

In den nachfolgenden Beispielen beziehen sich die Angaben der Ausbeute auf den bekannten Herstellprozeß nach DE-OS 23 64 039. Durch die erfindungsgemäßen Maßnahmen der Reindarstellung werden die gebildeten Carbonsäuren praktisch zu 100 % gewonnen. Die gefällten Kobaltsalze können in die Oxide überführt werden, aus denen in bekannter Weise die Kobaltcarbonyl-Katalysatoren hergestellt werden.

## Beispiel 1

In einem mit einem Rührer versehenen Druckautoklaven werden 3,5 kg Benzylchlorid, 2,16 kg Acetamid, 1,72 kg Natriumhydrogencarbonat, 4 Liter Wasser, 4 Liter Methylisobutylketon und 8 kg einer 13 Gew.-%igen Lösung von Dikobaltoctacarbonyl in Methylisobutylketon eingefüllt. Nach Zugabe eines Gemisches von CO und $H_2$ (Volumenverhältnis 1:1) bis zu einem Druck von 180 bar wird auf 110 $^0$C aufgeheizt. Ab einer Temperatur von 80 $^0$C findet eine rasche Aufnahme statt. Sobald kein Druckabfall mehr eintritt, wird abgekühlt und der restliche $CO/H_2$ abgeblasen. Die Reaktionsmischung wird mit 0,95 kg Oxalsäure, gelöst in 10 l Wasser, bei 80 $^0$C versetzt. Nach einer Stunde wird die Suspension gekühlt und mit einer 50 Gew.-%igen Natronlauge neutralisiert (pH 7,0).

Nach der Filtration und Trocknung erhält man 1,1 kg Kobaltoxalat. Aus dem Filtrat wird anschließend die wässrige Phase getrennt und mit konzentrierter HCl bei einer Temperatur zwischen 50 - 80 $^0$C bis pH1 versetzt. Nach dem Abkühlen, Filtration und Trocknung, erhält man 3,7 kg N-Acetyl-phenylalanin.

GC Reinheit ca. 98 %
Kobaltgehalt      40 ppm
Ausbeute          66,5 %, d.h. praktisch 100% des gebildeten
                  Acetyl-phenylalanins.

Aus der abgetrennten organischen Phase wird das Methylisobutylketon durch Destillation in einer Reinheit von über 99 % abgetrennt und bei der Reaktion wieder eingesetzt.

## Beispiel 2

Die Reaktion nach Beispiel 1 wird wiederholt, jedoch wird das Kobalt des Katalysators unter Verwendung von 1012 g Ameisensäure, anstelle von Oxalsäure, vollständig gefällt. Nach Abkühlen und Trocknung erhält man 696 g Kobaltformiat. Nach Ansäuern wie im Beispiel 1, jedoch mit Schwefelsäure, wird die festgestellte Menge des Zielprodukts N-Acetyl-phenylalanin zu fast 100 % erhalten.

Beispiel 3 bis 7

Analog Beispiel 1, jedoch unter Zugabe von folgenden Basen bis zu einem pH-Wert von 7,0

| Beispiel | Base | Reinheit des N-Acetyl-phenylalanin |
|---|---|---|
| 3 | $KOH$ | 98,0 |
| 4 | $Na_2CO_3$ | 99,2 |
| 5 | $NaHCO_3$ | 99,0 |
| 6 | $LiOH$ | 97,5 |
| 7 | $Ca(OH)_2$ | 98,0 |

wird das enthaltene Kobalt als Oxalat vollständig abgetrennt und das N-Acetylphenylalanin praktisch frei von Kobalt in der genannten hohen Reinheit erhalten.

Patentansprüche

1. Verfahren zur Reindarstellung von Carbonsäuren aus Reaktionslösungen mit Gehalten von Kobaltcarbonyl-Katalysatoren, dadurch gekennzeichnet, daß man die Reaktionslösung

a) mit Monocarbonsäuren mit 1 bis 3 C-Atomen, Dicarbonsäuren mit 2 bis 3 C-Atomen oder deren wässrige Lösungen in mindestens äquivalenter Menge zum Kobalt des Katalysators versetzt

b) die Reaktionslösung durch Zugabe alkalischer Mittel neutralisiert und das Kobaltsalz der nach a) zugegebenen Säure entfernt, und

c) die Carbonsäure aus der Reaktionslösung aufarbeitet.

2. Verfahren nach Anspruch 1, dadurch gekennzeichnet, daß zur Aufarbeitung der Reaktionslösung, die organische Phase nach Neutralisation mit alkalischen Mitteln von der wässrigen Phase, welche das Zielprodukt enthält, getrennt wird.

3. Verfahren nach Anspruch 2, dadurch gekennzeichnet, daß aus der wässrigen Phase das Zielprodukt durch Ansäuern gefällt und isoliert wird.

4. Verfahren nach einem der Ansprüche 1 bis 3, dadurch gekennzeichnet, daß die abgetrennte organische Phase in den Herstellprozeß zurückgeführt wird.